# EUROPEAN PATENT SPECIFICATION

(11) **EP 4 371 530 B1**
(45) Date of publication and mention of the grant of the patent: **03.06.2026**
(21) Application number: 23809943.6
(22) Date of filing: 17.08.2023
(51) Int. Cl.: A61F 2/24

(54) **VALVE IMPLANTATION DEVICE ASSISTED BY CARDIAC ISOLATION DEVICE**
DURCH EINE HERZISOLIERUNGSVORRICHTUNG UNTERSTÜTZTE KLAPPENIMPLANTATIONSVORRICHTUNG
DISPOSITIF D'IMPLANTATION DE VALVE ASSISTÉE PAR DISPOSITIF D'ISOLATION CARDIAQUE

(30) Priority: 26.09.2022 CN 202211177406
(43) Date of publication of application: 22.05.2024
(73) Proprietor: Nanjing Saint Medical Technology Co., Ltd., Nanjing, Jiangsu 210061 (CN)
(72) Inventor: MA, Chenming, Nanjing, Jiangsu 210061 (CN); QIN, Xiang, Nanjing, Jiangsu 210061 (CN); LI, Jiaxian, Nanjing, Jiangsu 210061 (CN); SHEN, Qilin, Nanjing, Jiangsu 210061 (CN)
(74) Representative: Gille Hrabal Partnerschaftsgesellschaft mbB Patentanwälte
(86) International application number: PCT/CN2023/113540
(87) International publication number: WO 2024/066785

(56) References cited:
- CN-A- 106 214 289
- CN-A- 110 944 599
- CN-A- 111 110 399
- CN-A- 112 438 825
- CN-A- 112 438 826
- CN-A- 113 730 034
- CN-U- 206 777 351
- DE-A1- 102013 017 750
- US-A1- 2014 031 928

## Description

### TECHNICAL FIELD

The present disclosure relates to the technical field of medical instruments, and in particular to a valve implantation device with the assistance of a heart isolation device.

### BACKGROUND

Valvular insufficiency is a common cardiac valve disease, mainly including two major categories: degenerative valvular insufficiency and functional valvular insufficiency. Degenerative diseases are mostly pathological changes of sub-valvular structures, mainly including rupture of chordae tendinca and/or valve leaflet abnormalities. Many functional diseases are secondary to ventricular dilatation and papillary muscle abnormalities caused by other diseases.

Current interventional therapies for atrioventricular insufficiency are less traumatic than surgery, thus receiving a lot of attention. However, clinical results have shown that there is no ideal product for interventional atrioventricular valve replacement. The main reasons are as follows. First, the structure and cause of disease of the atrioventricular valve are caused by various reasons, the insufficiency of most patients may have various causes or various structural problems. Only solving the implantation of a new valve cannot improve other structures. For example, in the scheme disclosed in patent No. CN102639179B, only a valve leaflet is replaced, which does not have a good effect on patients with other causes of impaired ventricular functions. Second, general atrioventricular valves are relatively large and need to be replaced by a stent made from a large number of metals. A too large stent causes high difficulty in operation and damages to heart structures. Third, valve anchorage in the prior art mostly depends on a supporting force of the metal stent. A valve moves after the implantation in some patients. This is because the anchorage is not stable, and a release height of the valve cannot be accurately controlled. CN 112 438 826 A discloses a valve prosthesis device to be implanted into a heart, that has a ventricular volume reducing component that is fixed to valve housing and is released in left ventricle and used to improve left ventricular enlargement caused by mitral valve regurgitation.

In view of the above, there is an urgent need for a heart valve implantation device that can improve the ventricular function simultaneously, achieve anchorage without a metal, and accurately release the height of the valve.

### SUMMARY

Provided is a valve implantation device with the assistance of a heart isolation device, which includes a valve, a heart isolation device, and a valve connection device, wherein the heart isolation device includes an isolation device body; the isolation device body is provided with a positioning part; the isolation device body is fixed on a heart tissue through the positioning part; the valve connection device comprises a first connection part; one side of the first connection part is connected with the valve; the isolation device body is provided with a second connection part; and the first connection part and the second connection part are connected in a ventricle during implantation of the valve.

Further, the positioning part on the isolation device body is of a thorn-like structure, which is located at a position where the isolation device body is in contact with the heart tissue, and can penetrate into the heart tissue to achieve fixation; and the thorn-like structure is oriented in any direction, preferably in an upward direction, a downward direction, or a combination of both the upward direction and the downward direction.

Further, the isolation device body further includes a fixing structure; and the fixing structure is arranged below the isolation device body and is connected to a ventricular tissue to stably position the isolation device body.

Further, the fixing structure on the heart isolation device is connected to the isolation device body by a tether or a suture; the fixing structure is located at the cardiac apex; the fixing structure comprises a structure that penetrates into the heart tissue, including a hook-like or thorn-like structure; the fixing structure is fixed on an inner wall of the cardiac apex; and the heart isolation device body is tensioned by the tether or the suture to provide reliable anchorage for the isolation device body.

Further, the first connection part connected to the valve is fixedly connected to the second connection part on the isolation device body by mechanical connection, preferably threaded connection, snap-in connection, buckle connection, rotational clamping connection, or knotted and bundled connection.

Further, the second connection part of the isolation device body includes a bulge structure; the first connection part connected to the valve has an indent structure and a buckle and/or a clamping slot; during connection, the bulge structure extends into the first connection part, and an edge of the bulge structure is fit connection wit the buckle and/or the clamping slot, so that fixed connection is achieved, and the disconnection is prevented; and preferably, the first connection part is connected to the valve by a suture. As another solution of the present disclosure, the second connection part includes a bulge structure; the first connection part is a rope or a suture; and during connection, the rope or the suture is knotted on the bulge structure by a knotting mechanism, so that the rope or the suture is fixedly connected to the bulge structure.

The valve implantation device according to the present disclosure further includes a delivery system, wherein the delivery system includes a first delivery system and a second delivery system; the first delivery system is configured to deliver the heart isolation device into the ventricle; and the second delivery system ins configured to deliver the valve.

The beneficial effects of the present disclosure are as follows:
The heart isolation device in the technical solutions of the present disclosure can improve the cardiac function before or synchronously during the implantation of the valve. Lesions of a valve annulus plane are treated, and lesions outside the valve annulus plane are also treated. The effectiveness of an operation is increased, and the type of patients is expanded.

The present disclosure fixes the valve by the hook-type structure, and can reduce metal objects required for replacement for atrioventricular valves with large structures. The reduction of the metal objects can avoid potential injury to a heart valve structure and improve the operation safety. Meanwhile, a size of the delivery system required to load metal objects is reduced, and the safety of operation is improved.

The connection device for connecting the valve to the heart isolation device, provided by the present disclosure, can be permanently connected to the subsequently implanted valve and stably locate the valve, thereby accurately releasing the height of the valve and playing a role of preventing paravalvular leak and preventing injury to the valve annulus plane.

### BRIEF DESCRIPTION OF THE DRAWINGS

FIG. 1 is a schematic structural diagram of a valve implantation device according to the present disclosure;
FIGs. 2a and 2b are schematic structural diagrams of various embodiments of a fixing structure;
FIGs. 3a to 3c are schematic structural diagrams of various embodiments of an isolation device body;
FIGs. 4a to 4e show a first embodiment of mounting a valve by using an implantation device of the present disclosure;
FIGs. 5a to 5c are schematic structural diagrams of a first embodiment of a first connection part and a second connection part;
FIGs. 6a and 6b are schematic structural diagrams of a second embodiment of a first connection part and a second connection part;
FIGs. 7a to 7c are schematic structural diagrams of a third embodiment of a first connection part and a second connection part;
FIGs. 8a to 8d show a process of delivering a heart isolation device into a ventricle through a first delivery system; and
FIGs. 9a to 9e show a second embodiment of mounting a valve by using an implantation device of the present disclosure.

### DETAILED DESCRIPTION

The present disclosure will be further illustrated with reference to the following specific examples. It should be understood that these examples are merely intended to illustrate the present disclosure rather than limit the protection scope of the present disclosure. In addition, it should be understood that various changes or modifications may be made by those skilled in the art after reading the teachings of the present disclosure.

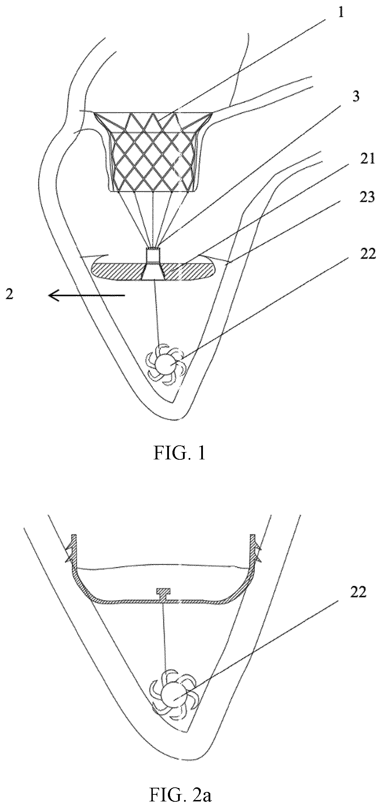

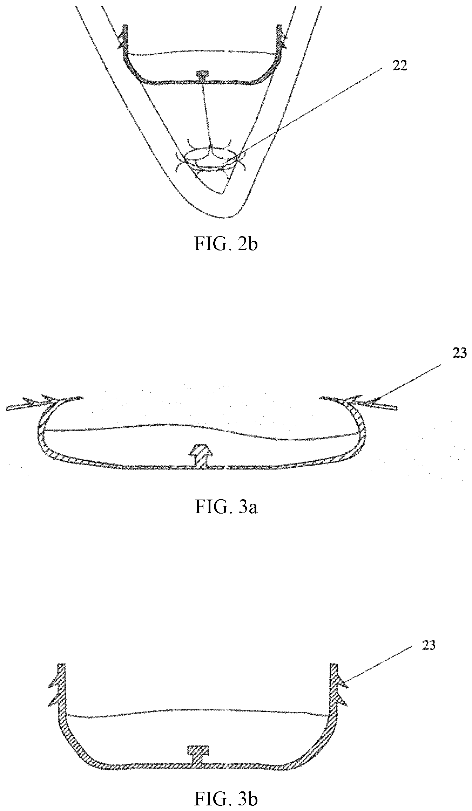

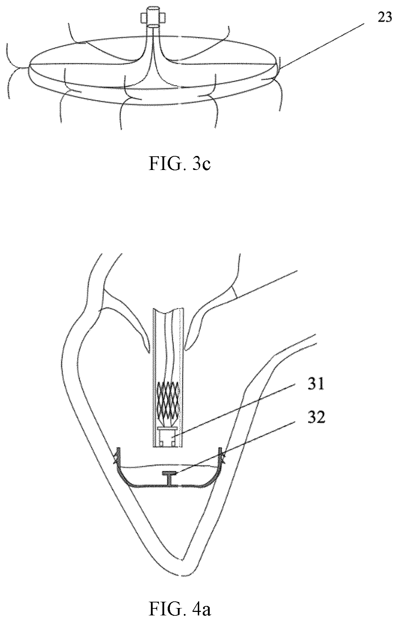

As shown in FIG. 1, a valve implantation device of the present disclosure includes a valve 1, a heart isolation device 2, and a valve connection device 3. As shown in FIGs. 2a and 2b, the heart isolation device 2 includes an isolation device body 21 and an optionally arranged fixing structure 22. The isolation device body is provided with a positioning part 23. The isolation device body 21 is fixed on a heart tissue through the positioning part 23. More specifically, the isolation device body 21 abuts against an inner wall of a ventricle through the positioning part 23. In this example, the positioning part is of a thorn-like structure, which can penetrate into the heart tissue to achieve fixation. Various embodiments can be adopted by changing the direction of a hook, as shown in FIGs. 3a to 3c.

The fixing structure 22 is arranged below the isolation device body 21. The fixing structure 22 is connected to the isolation device body 21 by a tether or a suture. The fixing structure 22 is located at the cardiac apex. The fixing structure 22 includes a structure that can penetrate into the heart tissue, including a hook-like or thorn-like structure, as shown in FIGs. 2a and 2b. On the one hand, the fixing structure 22 is fixed on an inner wall of the cardiac apex by penetrating into the heart tissue. On the other hand, the heart isolation device body 21 is reliably anchored by a tether or a suture.

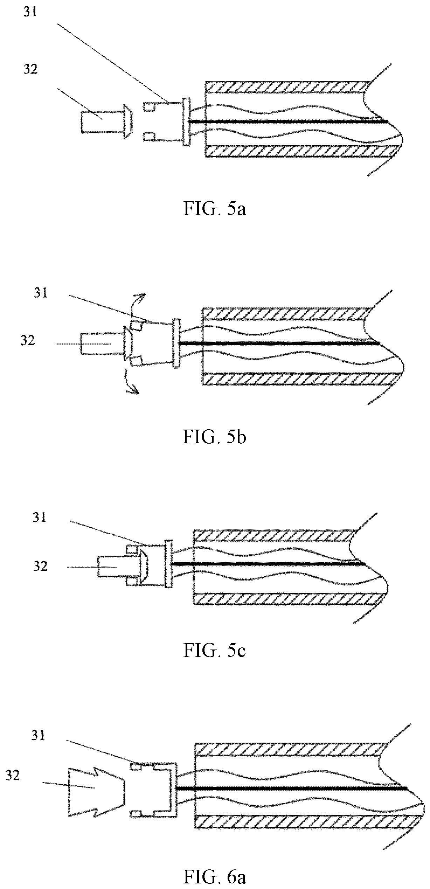

The valve connection device 3 includes a first connection part 31. One side of the first connection part 31 is connected to the valve 1 by a flexible structure, such as a suture and the like. The isolation device body 21 is provided with a second connection part 32. In this example, the second connection part 32 includes a bulge structure, which may be T-shaped as shown in FIG. 5a. The first connection part 31 has an indent structure and a buckle structure, as shown in FIGs. 5a to 5c. During connection, the bulge structure of the second connection part 32 extends into the first connection part 31, is in fit connection with a buckle, and is clamped and limited by the buckle, so that the first connection part 31 and the second connection part 32 are fixedly connected to effectively prevent disconnection.

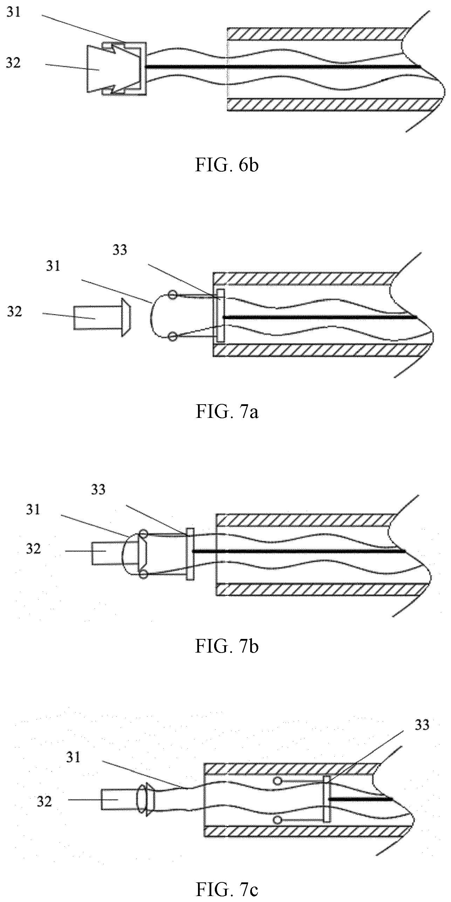

The first connection part 31 and the second connection part 32 can also adopt other embodiments, for example, as shown in FIGs. 6a and 6b. In this embodiment, a clamping slot is arranged in the first connection part 31. A bulge on the second connection part 32 is tower-shaped, an edge of which is hook-shaped. When the two connection parts are connected, the hook-shaped edge on the second connection part 32 is clamped in the clamping slot of the first connection part 31 to achieve their fixed connection.

As another embodiment of the present disclosure, the first connection part 31 is a suture, as shown in FIGs. 7a to 7c. During connection, the suture 31 is knotted on the bulge structure of the second connection part 32 by a knotting mechanism 33, so as to achieve their fixed connection.

The valve implantation device according to the present disclosure further includes a delivery system, wherein the delivery system includes a first delivery system and a second delivery system; the first delivery system is configured to deliver the heart isolation device into the ventricle; and the second delivery system is configured to deliver the valve.

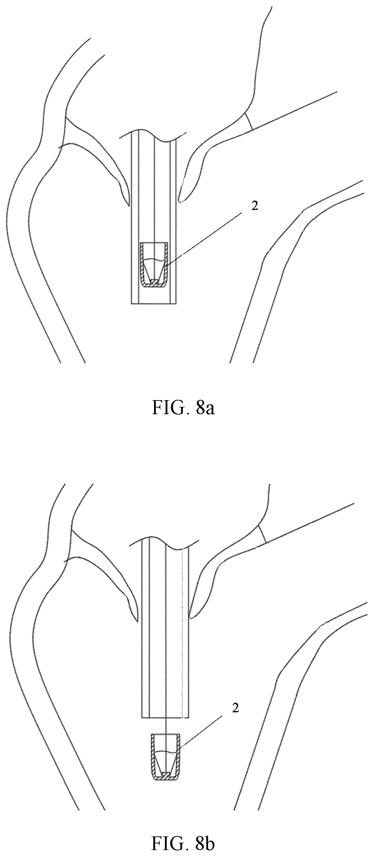

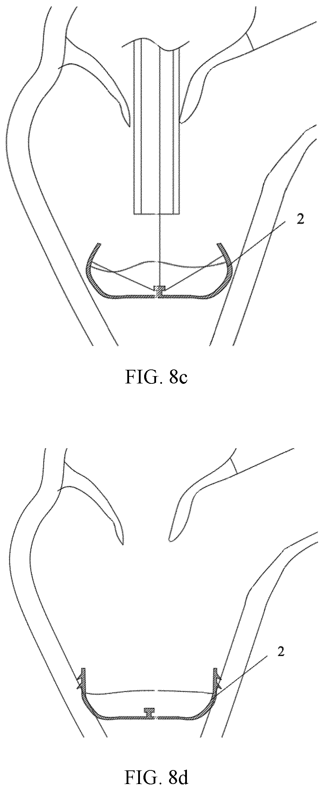

FIGs. 8a to 8d show a process of delivering a heart isolation device 2 into a ventricle through a first delivery system. The heart isolation device 2 is arranged at a distal end of the first delivery system. The heart isolation device 2 is released from the distal end and is delivered into a ventricle. The heart isolation device 2 is opened and shaped. After the heart isolation device 2 is fully opened, the first delivery system is withdrawn, and the thorn-like structure on the heart isolation device 2 penetrates into a heart tissue to play a role of positioning and fixing.

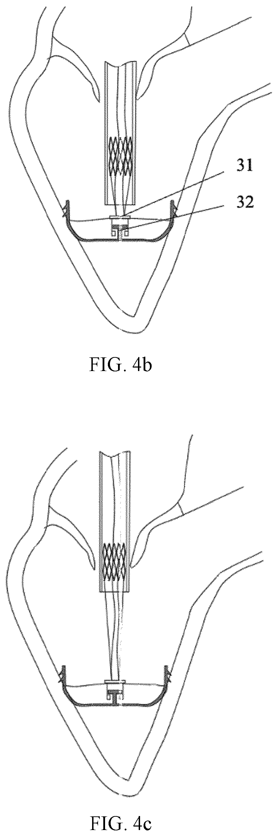

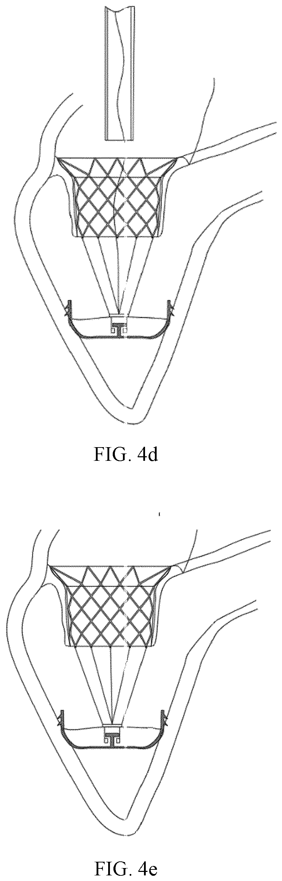

After the heart isolation device 2 is fixed, the valve 1 is delivered into the ventricle by the second delivery system and the valve 1 is mounted at a valve annulus, as shown in FIGs. 4a to 4e. The process specifically includes the following steps:
S1: mounting the valve 1 at the distal end of the second delivery system, wherein the valve 1 is connected with the first connection part 31;
S2: pushing the first connection part 31 to the isolation device 2 by arranging a push rod in the second delivery system, so that the first connection part 31 is connected to the second connection part 32;
S3: withdrawing the second delivery system, so that the valve 1 reaches the position of the valve annulus, at which the second delivery system is separated from the valve 1;
S4: adjusting the position and anchoring tightness of the valve 1 in a "wire control" manner, wherein optionally, the operation described above is implemented through an operating device at a proximal end of the delivery system, and after the position and anchoring tightness of the valve 1 have been adjusted, the suture on the valve 1 is knotted and fixed; and
S5: after the whole implantation of the valve 1 is completed, the second delivery system is withdrawn.

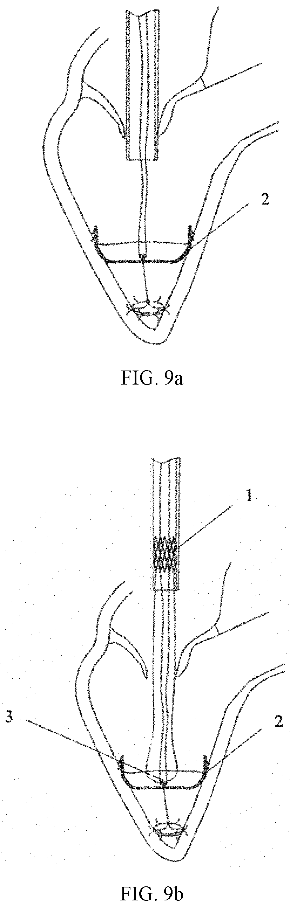

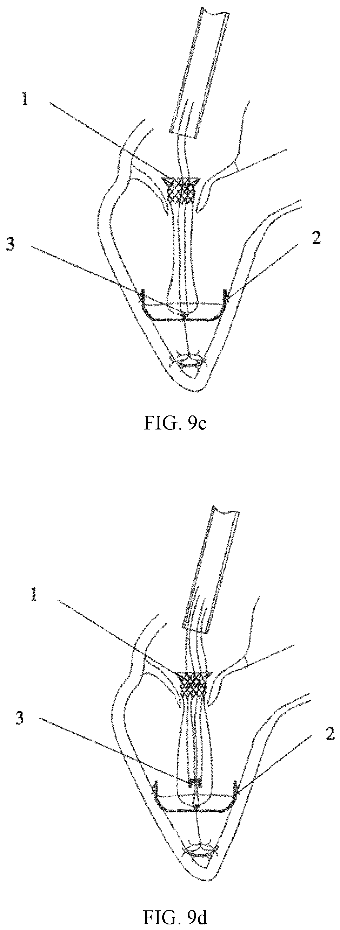

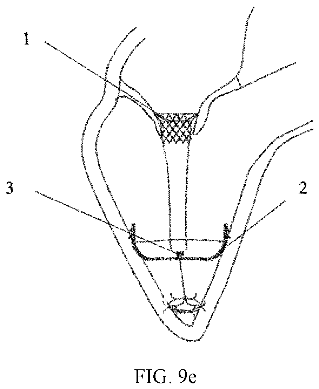

FIGs. 9a to 9e show another embodiment of the present disclosure, relating to another method for mounting a valve, including the following steps:
S1: after the heart isolation device 2 is fixed, connecting the suture to the second connection part 32 on the heart isolation device 2 using the second delivery system, preferably, by knotting;
S2: delivering the valve 1 to the distal end of the second delivery system in a wire control manner;
S3: releasing the valve 1 to an appropriate position at the valve annulus in a wire control manner;
S4, releasing the first connection part 31 to the position of the second connection part
32 in a wire control manner, and performing connection and fixing, thereby completing the positioning and permanent fixation of the valve 1; and
S5: withdrawing the delivery system and redundant sutures to complete the whole operation process.

## Claims

1. A valve implantation device with the assistance of a heart isolation device, comprising a valve (1), a heart isolation device (2), and a valve connection device (3), wherein the heart isolation device comprises an isolation device body (4); the isolation device body is provided with a positioning part (23); the isolation device body is fixed on a heart tissue through the positioning part; the valve connection device comprises a first connection part (31); one side of the first connection part is connected with the valve; the isolation device body is provided with a second connection part (32); and the first connection part and the second connection part are connected in a ventricle during implantation of the valve.

2. The valve implantation device as claimed in claim 1, wherein the positioning part on the isolation device body is of a thorn-like structure, which is located at a position where the isolation device body is in contact with the heart tissue, and can penetrate into the heart tissue to achieve fixation; and the thorn-like structure is oriented in any direction, preferably in an upward direction, a downward direction, or a combination of both the upward direction and the downward direction.

3. The valve implantation device as claimed in claim 2, wherein the isolation device body further comprises a fixing structure; and the fixing structure is arranged below the isolation device body and is connected to a ventricular tissue to stably position the isolation device body.

4. The valve implantation device as claimed in claim 3, wherein the fixing structure on the heart isolation device is connected to the isolation device body by a tether or a suture; the fixing structure is located at the cardiac apex; the fixing structure comprises a structure that penetrates into the heart tissue, including a hook-like or thorn-like structure; the fixing structure is fixed on an inner wall of the cardiac apex; and the heart isolation device body is tensioned by the tether or the suture to provide reliable anchorage for the isolation device body.

5. The valve implantation device as claimed in claim 4, wherein the first connection part connected to the valve is fixedly connected to the second connection part on the isolation device body by mechanical connection, preferably threaded connection, snap-in connection, buckle connection, rotational clamping connection, or knotted and bundled connection.

6. The valve implantation device as claimed in claim 5, wherein the second connection part of the isolation device body comprises a bulge structure; the first connection part connected to the valve has an indent structure and a buckle and/or a clamping slot; during connection, the bulge structure extends into the first connection part, and an edge of the bulge structure is fit connection with the buckle and/or the clamping slot, so that fixed connection is achieved, and the disconnection is prevented; and preferably, the first connection part is connected to the valve by a suture.

7. The valve implantation device as claimed in claim 5, wherein the second connection part comprises a bulge structure; the first connection part is a rope or a suture; and during connection, the rope or the suture is knotted on the bulge structure by a knotting mechanism, so that the rope or the suture is fixedly connected to the bulge structure.

8. The valve implantation device as claimed in any one of claims 1 to 7, further comprising a delivery system, wherein the delivery system comprises a first delivery system and a second delivery system; the first delivery system is configured to deliver the heart isolation device into the ventricle; and the second delivery system is configured to deliver the valve.

## Patentansprüche

1. Klappenimplantationsvorrichtung mit Unterstützung einer Herzisolationsvorrichtung, umfassend eine Klappe (1), eine Herzisolationsvorrichtung (2) und eine Klappenverbindungsvorrichtung (3), wobei die Herzisolationsvorrichtung einen Isolationsvorrichtungskörper (4) umfasst; wobei der Isolationsvorrichtungskörper mit einem Positionierungsteil (23) versehen ist; wobei der Isolationsvorrichtungskörper durch das Positionierungsteil an einem Herzgewebe fixiert wird; wobei die Klappenverbindungsvorrichtung ein erstes Verbindungsteil (31) umfasst; wobei eine Seite des ersten Verbindungsteils mit der Klappe verbunden ist; wobei der Isolationsvorrichtungskörper mit einem zweiten Verbindungsteil (32) versehen ist; und wobei das erste Verbindungsteil und das zweite Verbindungsteil während der Implantation der Klappe in einem Ventrikel verbunden sind.

2. Klappenimplantationsvorrichtung nach Anspruch 1, wobei das Positionierungsteil an dem Isolationsvorrichtungskörper eine dornenartige Struktur aufweist, die sich an einer Position befindet, an der der Isolationsvorrichtungskörper mit dem Herzgewebe in Kontakt vorliegt und in der Lage ist, in das Herzgewebe einzudringen, um eine Fixierung zu erzielen; und wobei die dornenartige Struktur in einer beliebigen Richtung ausgerichtet ist, vorzugsweise in einer Aufwärtsrichtung, einer Abwärtsrichtung oder einer Kombination von sowohl der Aufwärtsrichtung als auch der Abwärtsrichtung.

3. Klappenimplantationsvorrichtung nach Anspruch 2, wobei der Isolationsvorrichtungskörper ferner eine Fixierungsstruktur umfasst; und wobei die Fixierungsstruktur unterhalb des Isolationsvorrichtungskörpers angeordnet ist und mit einem Ventrikelgewebe verbunden ist, um den Isolationsvorrichtungskörper stabil zu positionieren.

4. Klappenimplantationsvorrichtung nach Anspruch 3, wobei die Fixierungsstruktur an der Herzisolationsvorrichtung durch eine Schnur oder einen Faden mit dem Isolationsvorrichtungskörper verbunden ist; wobei sich die Fixierungsstruktur an der Herzspitze befindet; wobei die Fixierungsstruktur eine Struktur umfasst, die in das Herzgewebe eindringt, einschließlich einer hakenartigen oder dornenartigen Struktur; wobei die Fixierungsstruktur an einer Innenwand der Herzspitze fixiert wird; und wobei der Herzisolationsvorrichtungskörper durch die Schnur oder den Faden gespannt wird, um eine zuverlässige Verankerung für den Isolationsvorrichtungskörper bereitzustellen.

5. Klappenimplantationsvorrichtung nach Anspruch 4, wobei das erste mit der Klappe verbundene Verbindungsteil mit dem zweiten Verbindungsteil an dem Isolationsvorrichtungskörper durch eine mechanische Verbindung, vorzugsweise eine Gewindeverbindung, eine Schnappverbindung, eine Schnallenverbindung, eine Drehklemmverbindung oder eine geknotete und gebündelte Verbindung, fest verbunden ist.

6. Klappenimplantationsvorrichtung nach Anspruch 5, wobei das zweite Verbindungsteil des Isolationsvorrichtungskörpers eine Wölbungsstruktur umfasst; wobei das erste mit der Klappe verbundene Verbindungsteil eine Einrückungsstruktur und eine Schnalle und/oder einen Klemmschlitz aufweist; wobei sich die Wölbungsstruktur während der Verbindung in das erste Verbindungsteil erstreckt und ein Rand der Wölbungsstruktur mit der Schnalle und/oder dem Klemmschlitz in Passverbindung steht, sodass eine fixierte Verbindung erzielt wird und die Trennung verhindert wird; und wobei das erste Verbindungsteil vorzugsweise durch einen Faden mit der Klappe verbunden ist.

7. Klappenimplantationsvorrichtung nach Anspruch 5, wobei das zweite Verbindungsteil eine Wölbungsstruktur umfasst; wobei das erste Verbindungsteil ein Seil oder ein Faden ist; und während der Verbindung das Seil oder der Faden durch einen Verknotungsmechanismus an die Wölbungsstruktur geknotet wird, sodass das Seil oder der Faden fest mit der Wölbungsstruktur verbunden ist.

8. Klappenimplantationsvorrichtung nach einem der Ansprüche 1 bis 7, ferner umfassend ein Abgabesystem, wobei das Abgabesystem ein erstes Abgabesystem und ein zweites Abgabesystem umfasst; wobei das erste Abgabesystem konfiguriert ist, um die Herzisolationsvorrichtung in das Ventrikel abzugeben; und wobei das zweite Abgabesystem konfiguriert ist, um die Klappe abzugeben.

## Revendications

1. Dispositif d'implantation de valve avec l'assistance d'un dispositif d'isolement du cœur, comprenant une valve (1), un dispositif d'isolement du cœur (2) et un dispositif de connexion de valve (3), dans lequel le dispositif d'isolement du cœur comprend un corps de dispositif d'isolement (4) ; le corps de dispositif d'isolement est muni d'une partie de positionnement (23) ; le corps de dispositif d'isolement est fixé sur un tissu cardiaque par l'intermédiaire de la partie de positionnement ; le dispositif de connexion de valve comprend une première partie de connexion (31) ; un côté de la première partie de connexion est connecté à la valve ; le corps de dispositif d'isolement est muni d'une deuxième partie de connexion (32) ; et la première partie de connexion et la deuxième partie de connexion sont connectées dans un ventricule lors de l'implantation de la valve.

2. Dispositif d'implantation de valve selon la revendication 1, dans lequel la partie de positionnement sur le corps de dispositif d'isolement est une structure en forme d'épine, située au niveau d'une position où le corps de dispositif d'isolement est en contact avec le tissu cardiaque, et peut pénétrer dans le tissu cardiaque pour assurer la fixation ; et la structure en forme d'épine est orientée dans une quelconque direction, de préférence dans une direction vers le haut, une direction vers le bas, ou une combinaison de la direction vers le haut et de la direction vers le bas.

3. Dispositif d'implantation de valve selon la revendication 2, dans lequel le corps de dispositif d'isolement comprend en outre une structure de fixation ; et la structure de fixation est agencée sous le corps de dispositif d'isolement et est connectée à un tissu ventriculaire pour positionner de manière stable le corps de dispositif d'isolement.

4. Dispositif d'implantation de valve selon la revendication 3, dans lequel la structure de fixation sur le dispositif d'isolement du cœur est connectée au corps de dispositif d'isolement par une attache ou une suture ; la structure de fixation est située au niveau de l'apex du cœur ; la structure de fixation comprend une structure qui pénètre dans le tissu cardiaque, y compris une structure en forme de crochet ou d'épine ; la structure de fixation est fixée sur une paroi interne de l'apex du cœur ; et le corps de dispositif d'isolement du cœur est tendu par l'attache ou la suture afin de fournir un ancrage fiable au corps de dispositif d'isolement.

5. Dispositif d'implantation de valve selon la revendication 4, dans lequel la première partie de connexion connectée à la valve est connectée de manière fixe à la deuxième partie de connexion sur le corps de dispositif d'isolement par une connexion mécanique, de préférence une connexion filetée, une connexion à enclenchement, une connexion à boucle, une connexion à serrage rotatif ou une connexion nouée et groupée.

6. Dispositif d'implantation de valve selon la revendication 5, dans lequel la deuxième partie de connexion du corps de dispositif d'isolement comprend une structure renflée ; la première partie de connexion connectée à la valve présente une structure en creux et une boucle et/ou une fente de serrage ; lors de la connexion, la structure renflée s'étend jusque dans la première partie de connexion, et un bord de la structure renflée est ajusté à la boucle et/ou à la fente de serrage, de manière à obtenir une connexion fixe et à empêcher la déconnexion ; et de préférence, la première partie de connexion est connectée à la valve par une suture.

7. Dispositif d'implantation de valve selon la revendication 5, dans lequel la deuxième partie de connexion comprend une structure renflée ; la première partie de connexion est une corde ou une suture ; et lors de la connexion, la corde ou la suture est nouée sur la structure renflée par un mécanisme de nouage, de sorte que la corde ou la suture est connectée de manière fixe à la structure renflée.

8. Dispositif d'implantation de valve selon l'une quelconque des revendications 1 à 7, comprenant en outre un système de distribution, dans lequel le système de distribution comprend un premier système de distribution et un deuxième système de distribution ; le premier système de distribution est configuré pour distribuer le dispositif d'isolement du cœur dans le ventricule ; et le deuxième système de distribution est configuré pour distribuer la valve.
